# EUROPEAN PATENT SPECIFICATION

(11) **EP 2 637 927 B1**
(45) Date of publication and mention of the grant of the patent: **03.01.2018**
(21) Application number: 11834865.5
(22) Date of filing: 11.10.2011
(51) Int. Cl.: A61B 17/132, A41D 13/05, A63B 71/10

(54) **DEVICE TO REDUCE SLOSH ENERGY ABSORPTION EFFECTS BY REDUCING BLOOD FLOW FROM THE CRANIUM**
VORRICHTUNG ZUR REDUZIERUNG VON SLOSH-ENERGIEABSORPTIONSEFFEKTEN MITTELS REDUZIERUNG DES BLUTFLUSSES AUS DEM SCHÄDELS
DISPOSITIF DE RÉDUCTION DES EFFETS D'ABSORPTION DE L'ÉNERGIE DE BALLOTTEMENT GRÂCE À LA RÉDUCTION DE L'ÉCOULEMENT SANGUIN DEPUIS LE CRÂNE

(30) Priority: 01.02.2011 US 931415; 29.04.2011 US 201161518117 P
(43) Date of publication of application: 18.09.2013
(73) Proprietor: Thornhill Research Inc, Toronto, ON M5G 2E8 (CA); TBI Innovations, LLC, Richmond, IN 47374 (US)
(72) Inventor: SMITH, David, Richmond IN 47374 (US); FISHER, Joseph, Arnold, Toronto, ON M5G 2E8 (CA)
(74) Representative: Schiweck, Weinzierl & Koch Patentanwälte Partnerschaft mbB
(86) International application number: PCT/US2011/055783
(87) International publication number: WO 2012/054262

(56) References cited:
- WO-A1-98/46144
- FR-A- 719 730
- US-A- 2 271 927
- US-A- 2 676 586
- US-A- 3 765 412
- US-A- 4 628 926
- US-A- 5 403 266
- US-A- 5 643 315
- US-A1- 2011 028 934
- US-B2- 7 100 251
- VANNUCCI RC ET AL.: 'Carbon dioxide protects the perinatal brain from hypoxic-ischemic damage: an experimental study in the immature rat.' PEDIATRICS. vol. 95, no. 6, June 1995, pages 868 - 74, XP008171602

## Description

### TECHNICAL FIELD

The present disclosure is generally related to methods and devices for restricting blood flow from the head, thereby reducing SLOSH energy absorption and neural tissue damage.

### BACKGROUND

Traumatic brain injury (TBI) continues to be one of the most common causes of death and morbidity in persons under age 45, even in western societies. A reported 1.7 million people suffer from TBI annually in the United States alone, resulting in an estimated per annum total cost of over $60 billion. Historically, prevention of skull and brain injury has focused on the use of helmets as external cranial protection. This approach is
fundamentally flawed as irrespective of the benefits, helmets have provided benefit for only major penetrating brain injuries and skull fractures. These occur in a very small fraction of head injuries in civilian sphere. Military statistics have shown that even on the battlefield, less than 0.5% of TBI is from a penetrating object. However, both military personnel and athletes are subjected to high velocity, acceleration-deceleration mechanisms that are not mitigated by helmets and lead to concussive injury to the brain. In large part, the human brain's relative freedom of movement within the cranial cavity predisposes to both linear and rotational force vectors, with resultant energy absorption resulting in cellular disruption and dysfunction, sometimes with delayed cell death.

The skull and spinal canal contains only nervous tissue, connective tissue and fat cells and their interstitium, blood, and cerebrospinal fluid (CSF). These fluid contents do not completely fill the rigid container delimited by the skull and bony spinal canal, leaving a 'reserve volume'. The change in volume inside a container for a given change in pressure is termed 'compliance'. Increases in volume of the contents of the skull and bony spinal canal, within the range of reserve volume, occur at low container pressures (due to the high compliance of the system). In the presence of reserve volume, as is seen in a normal physiologic state, acceleration to the skull can result in a differential acceleration between the skull and its contents. As a consequence, the brain and fluids collide with themselves and the inside of the skull. Considering the semi-solid properties of the mammalian brain, this effect is referred to as "slosh".

While helmets are effective in preventing the infrequent penetration or fracture of the skull, they have little ability to limit slosh effects. Mitigating slosh by filling the reserve volume (exhausting compliance) can, therefore, significantly reduce the propensity for differential motion between the skull and its contents, and between the various contents of the skull. By mitigating slosh, an accelerating force to the skull would tend to move the skull and its contents in unison, preventing collisions amongst intracranial contents and, therefore, avoiding brain kinetic, acoustic, thermal, and vibrational energy absorption.

In an attempt to mitigate intracranial slosh it is recognized that the single intracranial compartment that is most amenable to rapid, reversible change in volume and pressure is the blood space. The simplest and most rapid means of increasing the blood compartment is to inhibit its outflow by mechanically obstructing the draining veins in the neck.

Standard prophylactic measures designed to protect the brain against injury in the case of head trauma have hitherto included only various helmets. Helmets are primarily designed to protect the skull from penetrating injuries and fractures, but less so from pathological movements of the brain, exemplified by the classic cerebral concussion. It has been shown that bracing the brain inside the skull by increasing the intracranial blood volume and thereby the range of compliance, reduced neuronal damage from a standardized cranial impact. The analogy in automobiles would be the difference between placing additional armor on the outside of the car, and wearing a seat belt. The additional external armor of the vehicle improves the external integrity of the vehicle but completely ignores the interior compartment in which the passengers would still be subjected to extreme forces due to rapid differential acceleration. The seatbelt greatly diminishes these forces as the vehicle and its passengers now move in the same direction.

US Patent 2,676,586 discloses a device for preventing blacking-out of pilots during sudden changes in direction of an airplane traveling at high speed. The device includes a collar carrying inflatable pads which are so positioned that they are adapted to apply pressure to veins carrying blood from the head.

US Patent 5,643,315 discloses a system for applying pressure to a wound site in order to prevent bleeding. The system comprises a unit that is primarily made from a pliable, non-stretchable support unit made from a fabric, and which supports a pressure-producing device such as a spring or lever system and particularly a balloon or an expandable unit in order to press the wound site to provide wound therapy and prevent bleeding.

PCT application WO 98/46144 discloses a hemostasis device and a method for closing wounds by the application of pressure. The device includes one or more inflatable bladders which may be coupled to a portion of a patient's body in order to apply pressure to a wound site upon inflation.

US Patent 2,271,927 discloses a tourniquet suitable for retarding the flow of blood and lymph secretions in the tubular, superficial trunks and chains of minor vessels located adjacent the surface of the body.

French Patent No. 719,730 discloses a compression device against seasickness in the form of a rubber strip having two round balls which is to be worn around the neck in order to regulate the flow of blood in the higher nervous centers.

US patent application US 2011/0028934 A1 discloses devices and methods for achieving hemostasis in patients who have received skin-penetrating wounds to the body in regions such as the shoulder, pelvis, neck, or groin, where standard bandages are difficult to apply and where large blood vessels exist which can hemorrhage severely. The devices utilize packing pillows that are held in place by rigid structures that can cause the packing pillows to be brought into the wounds and be held in place while the packing pillows are inflated to fill the wounds, prevent bleeding, and tamponade hemorrhaging large blood vessels exposed therein.

To date, no effective pharmacological treatment has been discovered for TBI. Although new compounds, such as progesterone, are undergoing clinical trials, the ability to intervene and mitigate the secondary injury cascade of TBI is elusive. Preventative measures against TBI one day may provide some degree of protection, but thus far conceptually have only focused upon external forces and factors. There is a potential of reducing neuronal damage by administration of omega-3 fatty acids, including
docosahexaenoic acid (DHA) both prophylactically and following TBI.

### SUMMARY

Traumatic brain injury (TBI) remains a devastating condition for which traditionally extra-cranial protection has been utilized in the form of helmets. Although headgear is effective in preventing the most devastating intracranial injuries, penetrating injuries, and skull fractures, it is limited in its ability to prevent concussions. The present invention as claimed relates to a collar device as recited in the independent claims. By use of the claimed collar device, the internal jugular vein (UN) is mildly occluded to increase cerebral blood volume and decrease the intracranial compliance. This results in a reduction of the differential acceleration between the skull and its contents, less propensity for brain and fluid movement inside the skull, resulting in less shearing and tearing forces and less energy absorption by the contents, all resulting in less traumatic axonal and glial injury.

One aspect of the disclosure encompasses embodiments of a device to reduce SLOSH energy absorption in an animal or human subject by reducing the flow of one or more neck veins by compressing at least one of said vessels, wherein the device can comprise a collar configured to at least partially encircle the neck of an animal or human subject, and at least one region inwardly directed to contact the neck when encircled by the collar, thereby applying a localized pressure to a neck vein.

In some embodiments of this aspect of the disclosure, the at least one region inwardly directed to contact the neck can be selected from the group consisting of: a protuberance, a stud, and a thickened region of the collar, and wherein the protuberance, the stud, and the thickened region of the collar can be rigid, semi-rigid, or inflatable.

In some embodiments of this aspect of the disclosure, the at least one region inwardly directed to contact the neck can be disposed on said collar to exert pressure in the area of an internal jugular vein when the neck of an animal or human subject is inserted in said collar.

In some embodiments of this aspect of the disclosure, the collar can be elastic.

In some embodiments of this aspect of the disclosure, the collar size and tension thereof can be adjustable.

In some embodiments of this aspect of the disclosure, the device can further comprise one or more breakaway release mechanisms.

In some embodiments of this aspect of the disclosure, the device can further comprise a monitoring device, a recording device, a communicating device, or any combination thereof.

In some embodiments of this aspect of the disclosure, at least one region of the device inwardly directed to contact the neck of a subject can be formed by inflation of a region of the collar, and wherein the device optionally further comprises a pump to inflate the inflatable protuberance, or any region of said device, and optionally a source of pressurized gas or fluid for inflation thereof.

In some embodiments of this aspect of the disclosure, the device can further comprise a release valve to regulate the pressure in said collar.

Another aspect of the disclosure encompasses embodiments of a method of increasing the intracranial pressure of an animal or human subject comprising: (i) encircling the neck of an animal or human subject with a collar, wherein said collar has at least one region inwardly directed to contact the neck of an animal or human subject; (ii) positioning the at least one region inwardly directed to contact the neck on a region of the neck overlying a neck vein carrying blood from the intracranial cavity of the subject; and (iii) applying pressure to the neck vein by pressing the at least one region inwardly directed to contact the neck onto the surface of the neck, thereby restricting blood flow egressing the intracranial cavity of the subject, thereby increasing the intracranial pressure and or volume of the subject.

In some embodiments of this aspect of the disclosure, the method can further comprise the step of increasing the pC02 of the intracranial cavity, thereby reducing the effect of intracranial sloshing on the blood of the animal or human subject.

### BRIEF DESCRIPTION OF THE DRAWINGS

Further aspects of the present disclosure will be more readily appreciated upon
review of the detailed description of its various embodiments, described below, when taken in conjunction with the accompanying drawings.
Fig. 1 is a graph illustrating the change in intracranial pressure (ICP) as a consequence of IJV compression, p-value <0.01.
Fig. 2 is a graph illustrating the change in intraocular pressure (IOP) as a consequence of IJV compression, p-value 0.01.
Fig. 3 is a graph showing a representative tracing of physiologic change seen in intracranial pressure (ICP) and intraocular pressure (IOP) over a fifteen minute period caused by the application (arrow on left) and removal of IJV compression (arrow on right). Of note is the rapid response seen in both IP and IOP following IJV compression as well as the duration for which these changes are sustained.
Fig. 4A is a digital image of corticospinal tracts stained for APP post-injury without application of the IJV compression device according to the disclosure.
Fig. 4B is a digital image of corticospinal tracts stained for APP post-injury with application of the IJV compression device according to the disclosure.
Fig. 5 is a graph illustrating the effect of IJV compression on axonal injury as indicated by APP staining, p-value <001.

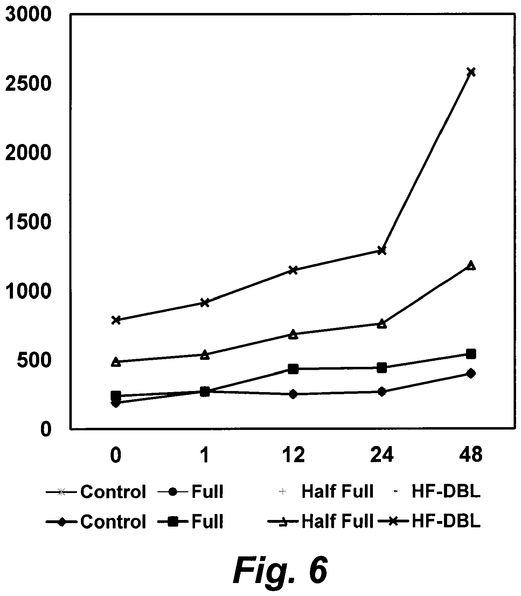

Fig. 6 is a graph illustrating blood LDH levels in blood samples at 0, 1, 12, 24, and 48 hrs after gas blast exposure. Vials containing 8ml (Control, One-half Full) and 16ml (Full) of heparinized blood were subject to a single 15ml C0₂ blast, and a One-half Full vial containing 8ml of heparinized blood was subject to a double 15ml C0₂ blast.

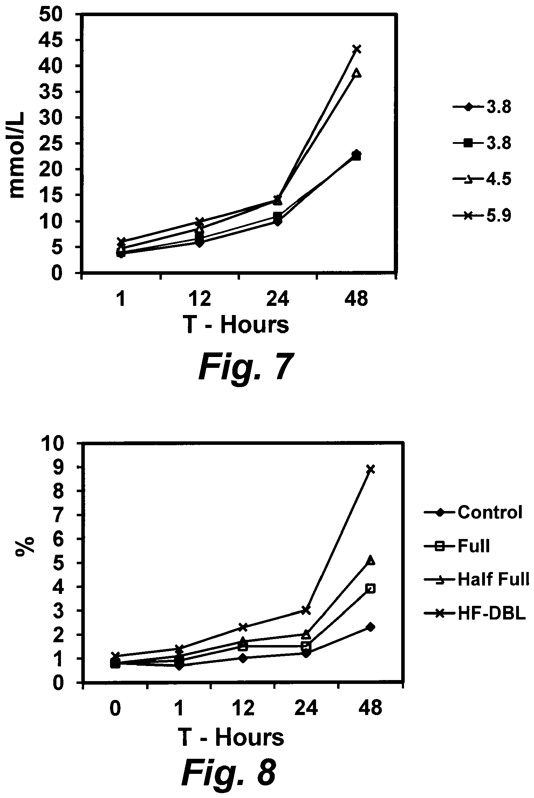

Fig. 7 is a graph illustrating blood potassium levels in blood samples at 0, 1, 12, 24, and 48 hrs after gas blast exposure. Vials containing 8ml (Control, One-half Full) and 16ml (Full) of heparinized blood were subject to a single 15ml C0₂ blast, and a One-half Full vial containing 8ml of heparinized blood was subject to a double 15ml C0₂ blast.
Fig. 8 is a graph illustrating blood methemoglobin levels in blood samples at 0, 1, 12, 24, and 48 hrs after gas blast exposure. Vials containing 8ml (Control, One-half Full) and 16ml (Full) of heparinized blood were subject to a single 15ml C0₂ blast, and a One-half Full vial containing 8ml of heparinized blood was subject to a double 15ml C0₂ blast.

The drawings are described in greater detail in the description and examples below.

The details of some exemplary embodiments of the methods and systems of the present disclosure are set forth in the description below.

### DETAILED DESCRIPTION

Where a range of values is provided, it is understood that each intervening value, to the tenth of the unit of the lower limit unless the context clearly dictates otherwise, between the upper and lower limit of that range and any other stated or intervening value in that stated range, is encompassed within the disclosure. The upper and lower limits of these smaller ranges may independently be included in the smaller ranges and are also encompassed within the disclosure, subject to any specifically excluded limit in the stated range. Where the stated range includes one or both of the limits, ranges excluding either or both of those included limits are also included in the disclosure.

Unless defined otherwise, all technical and scientific terms used herein have the same meaning as commonly understood by one of ordinary skill in the art to which this disclosure belongs. Although any methods and materials similar or equivalent to those described herein can also be used in the practice or testing of the present disclosure, the preferred methods and materials are now described.

As will be apparent to those of skill in the art upon reading this disclosure, each of the individual embodiments described and illustrated herein has discrete components and features which may be readily separated from or combined with the features of any of the other several embodiments without departing from the scope of the present disclosure. Any recited method can be carried out in the order of events recited or in any other order that is logically possible.

Embodiments of the present disclosure will employ, unless otherwise indicated, techniques of medicine, organic chemistry, biochemistry, molecular biology, pharmacology, and the like, which are within the skill of the art. Such techniques are explained fully in the literature.

It must be noted that, as used in the specification and the appended claims, the singular forms "a," "an," and "the" include plural referents unless the context clearly dictates otherwise. Thus, for example, reference to "a support" includes a plurality of supports. In this specification and in the claims that follow, reference will be made to a number of terms that shall be defined to have the following meanings unless a contrary intention is apparent.

As used herein, the following terms have the meanings ascribed to them unless specified otherwise. In this disclosure, "comprises," "comprising," "containing" and "having" and the like can have the meaning ascribed to them in U.S. Patent law and can mean " includes," "including," and the like; "consisting essentially of or "consists essentially" or the like, when applied to methods and compositions encompassed by the present disclosure refers to compositions like those disclosed herein, but which may contain additional structural groups, composition components or method steps (or analogs or derivatives thereof as discussed above). Such additional structural groups, composition components or method steps, etc., however, do not materially affect the basic and novel characteristic(s) of the compositions or methods, compared to those of the corresponding compositions or methods disclosed herein. "Consisting essentially of or "consists essentially" or the like, when applied to methods and compositions encompassed by the present disclosure have the meaning ascribed in U.S. Patent law and the term is open-ended, allowing for the presence of more than that which is recited so long as basic or novel characteristics of that which is recited is not changed by the presence of more than that which is recited, but excludes prior art embodiments.

### Description

When liquid in a tank or vessel experiences dynamic acceleration or deceleration, there will develop a variety of wave motions in the liquid. The oscillation of a fluid caused by external force, termed "sloshing", occurs in moving vessels containing liquid masses, such as trucks, aircraft, and liquid fueled rockets. This sloshing effect can be a severe problem in energy absorption, and vehicle stability and control. The present disclosure encompasses methods and apparatus for reducing slosh effects in living creatures, and in particular in the intracranial regions of the animal or human subject.

The mitigation of blast wave and collision damage is based largely on the principle of energy absorption of fluid-filled containers. As there becomes more room for movement of fluid within a vessel, more energy can be absorbed (SLOSH) rather than transmitted through the vessel. To reduce this energy absorption, one must attempt to more closely approximate elastic collisions. Elastic collisions are those that result in no net transfer of energy, chiefly, acoustic, kinetic, vibrational, or thermal (also stated as a coefficient of restitution (r) approximating 1.0). Various embodiments described below may locally alter, elevate, or temporarily maintain an altered physiology of an organism to reduce the likelihood of energy absorption through SLOSH whereby the coefficient of restitution (r) is increased. The coefficient of restitution (r) indicates the variance of an impacting object away from being a complete total elastic collision (an (r) of 1.0 = no energy transfer). Blast or energy absorption in an organism can be viewed as a collision of bodies and thus be defined by a transfer of energies through elastic or inelastic collisions. The mechanisms for biological fluids and molecules to absorb energy can thus be identified and the resultant means to mitigate that absorption can be achieved through several SLOSH reducing techniques. Dissipation of energies post blast is also potentiated through these techniques.

An effort to reduce the available space for movement of the brain by increasing cerebral blood volume can serve the purpose of mitigating Traumatic Brain Injury (TBI), red cell injury, or increasing orthostatic or G-tolerance through SLOSH mitigation at a cellular level (micro SLOSH) or organ level (macroSLOSH). Red blood cells (RBC) or erythrocytes are highly distensible and have a "sloshable" volume to surface area of only 60 percent. Distending or stiffening these erythrocytes can reduce SLOSH within the individual cells at a cellular (micro SLOSH) level and thus reduce energy absorption upon collision. Molecules themselves have a three dimensionality and can have a lack of cross-bridging providing for floppy conformational changes that can promote SLOSH. Several mechanisms disclosed can safely and reversibly alter the conformational state of certain structures, cells and molecules in the circulatory system that will then reduce energy absorption through SLOSH at a molecular (molecular SLOSH) level.

Co-elevating of the local C0₂ and hence lowering the pH environment of an organism can also serve to mitigate SLOSH. Raised inspired C0₂ (hypercapnia) can mitigate TBI through the reduction of macroSLOSH inside the cranium, but also has the ability to reduce the microSLOSH inside each individual RBC and reduce the molecular SLOSH of each individual hemoglobin molecule. Each of these physiology changes allows a better passage of imparted forces through the blood and brain tissues with less of the energy being absorbed. Within the brain's more than 150 cc of cerebral blood, there are more than 10¹² erythrocytes (1 trillion cells) that hypercapnia can potentiate to more closely approximate elastic collisions of cells and thus reducing the blast or collision energy absorption. Further, a hypercapnic state can also potentiate the collisions of all the hemoglobin molecules present in the cranium and body to be more elastic, thus reducing blast or collision energy absorption. There are 8 x 10¹² RBC in the human body, more than one trillion in the brain space at any one time. All of these cells are susceptible to SLOSH energy absorption, and that absorption would be reduced in the setting of hypercapnia. Further, there are about 2.4 x 10⁸ molecules of hemoglobin inside each RBC. Consequently, there are thus 1.9 x 10²² hemoglobin molecules which are able to absorb the energies of a blast. The SLOSH energy absorption of these molecules can be significantly reduced by hypercapnia altering the molecules to approximate more elastic collisions.

Hemoglobin is made up of four iron containing heme components and four globins that surround (pocket) each heme, and in essence waterproof these hemes. If the blast energies are absorbed by fluids and blood cells, they are preferentially absorbed by hemoglobin which is then conformationally altered to allow water to enter the heme-pocket leading to a rapid, catalytic oxidation to methemoglobin and superoxide. Superoxide is oxygen with an extra electron; methemoglobin is merely an oxyhemoglobin devoid of a superoxide. Without this extra electron, methemoglobin does not have the ability to carry or transfer oxygen (thus the brain suffocates), and in the case of blast lung, massive levels of methemoglobin have been recorded. The erythrocytes can slowly reduce methemoglobin back to functional hemoglobin but if this methemoglobin reductase reaction is not capable of diverting adequate electrons to counter this redox chemistry, spillover occurs into the oxidative damaging formation of superoxide, nitric oxide, peroxinitrite, etc. When an electron moves from one molecule to another, the donor molecule is thus oxidized while the receiving molecule is reduced (hence the term "redox"). For decades methylene blue has been used as the safe and well tolerated antidote for cyanide poisoning (and methemoglobinemias). It safely and dramatically facilitates the reductive pathways of methhemoglobin back into hemoglobin.

Hypercapnia not only pushes methylene blue into erythrocytes where it can be functional, but it also appears to actually drive methemoglobin reductase to more quickly convert methemoglobin back to hemoglobin. Further, the anti-oxidants (electron donors) ascorbic acid (vitamin C) and riboflavin are also driven into the erythrocyte by hypercapnia; These antioxidants are not useful for post blast or energy absorption outside of erythrocytes. A soldier or athlete can be given physiologic daily doses of Vitamin C, Riboflavin and methylene blue (not a vitamin) and upon triggering a need, hypercapnia will drive these cofactors into the erythrocyte where they can mitigate the after effects of blast energy absorption.

The present disclosure encompasses embodiments of a method to reduce SLOSH energy absorption through reduction of inelastic collisions in a fluid-containing organism wherein the method is one or more of reversibly increasing pressure, or volume within the organs or cells, or reversibly altering vascular, molecular, or cell wall stiffness or configuration within said organism. One embodiment of a method to increase the volume and/or pressure within the cranium can be by temporarily raising the pCO₂ in the body of the organism by way of altering the fractional percentage of CO₂ inspired by the organism or reducing its CO₂ elimination below that of CO₂ production by the body. Such a method can maintain the above hypercapnic inspired CO₂ levels to exceed baseline levels. The CO₂ is actively and instantly pumped into erythrocytes and after the external CO₂ delivery stops, the intracellular CO₂ levels may take hours to return to normal. These levels can be achieved and maintained by an externally imparted respiratory circuit which can modulate the fractional percentage of CO₂ inspired by the organism or reduce the rate of CO₂ elimination below that of CO₂ production by the body. The circuit could be one or more of a non-breathing circuit, a breathing circuit mask, or a breathing circuit capable of organizing exhaled gas so as to modulate the fractional percentage of CO₂ inspired by the organism (a range from 0.05 to 100% could be utilized) or net reduction of CO₂ elimination below that of CO₂ production by the body. The circuit can include a customizable re-breathing circuit whose dead space is adjustable based on an individual's weight and estimated tidal volume, and desired or optimized level of hypercapnia (a pCO₂ rise of at least 2 mm Hg above baseline levels would be optimum). The mask or vessel can incorporate one or several dead space channels or tubes that provide an inhale and exhale pathway that superimpose each other and thereby create mixing of inspired and expired gases. Alternatively, a source of fresh gas, potentially containing CO₂ can be supplemented when capnography (measurement of exhaled end-tidal CO₂), if utilized, so indicates. A re-breathing respiratory circuit may have one or more of the following: a mask or collecting vessel acting as an interface between the breathing circuit and the breathing passages which has one or multiple channels or tubes whose length or volume is rapidly adjustable to regulate the amount of dead space containing previously exhaled gas that an individual will re-breath for the express purpose of raising or modulating their local CO₂ level within their blood stream. The circuit may also contain a physiologically insignificant amount of CO₂ in communication with a valve to be delivered to the patient, a fresh gas reservoir in communication with the source of fresh gas flow for receiving fresh gas during exhalation, and a reserve gas supply containing CO₂ in communication with the exit port through the valve. Alternatively, a non-rebreathing circuit can be comprised of one or more of the following: a non-rebreathing valve preventing gas exhaled from the subject flowing into the circuit, a fresh gas source operative to supply a fresh gas containing physiologically insignificant amount of carbon dioxide to the subject through the non-rebreathing valve, and a reserved gas source operative to supply a reserved gas having a predetermined partial pressure of carbon dioxide to the subject, also through the inspiratory port of the non-rebreathing valve.

Venous blood returns to the heart from the muscles and organs partially depleted of oxygen and containing a full complement of carbon dioxide. Blood from various parts of the body is mixed in the heart (mixed venous blood) and pumped into the lungs via the pulmonary artery. In the lungs, the blood vessels break up into a net of small capillary vessels surrounding tiny lung sacs (alveoli). The vessels surrounding the alveoli provide a large surface area for the exchange of gases by diffusion along their concentration gradients. After a breath of air is inhaled into the lungs, it dilutes the CO₂ that remains in the alveoli at the end of exhalation. A concentration gradient is then established between the partial pressure of CO₂ (pCO₂) in the mixed venous blood (pvCO₂) arriving at the alveoli and the alveolar pCO₂. The CO₂ diffuses into the alveoli from the mixed venous blood from the beginning of inspiration (at which time the concentration gradient for CO₂ is established) until equilibrium is reached between the pCO₂ in blood from the pulmonary artery and the pCO₂ in the alveoli at some time during breath. The blood then returns to the heart via the pulmonary veins and is pumped into the arterial system by the left ventricle of the heart. The pCO₂ in the arterial blood, termed arterial pCO₂ (p_{A}CO₂), is then the same as was in equilibrium with the alveoli. When the subject exhales, the end of his exhalation is considered to have come from the alveoli and thus reflects the equilibrium CO₂ concentration between the capillaries and the alveoli. The pCO₂ in this gas is the end-tidal pCO₂ (p_{ET}CO₂). The arterial blood also has a pCO₂ equal to the pCO₂ at equilibrium between the capillaries and alveoli.

With each exhaled breath some CO₂ is eliminated and with each inhalation, fresh air containing minimal CO₂ (presently 0.04%) is inhaled and dilutes the residual equilibrated alveolar pCO₂, establishing a new gradient for CO₂ to diffuse out of the mixed venous blood into the alveoli. The rate of breathing, or ventilation (V_{E}), usually expressed in L/min, is exactly that required to eliminate the CO₂ brought to the lungs and establish an equilibrium between the alveolar PCO₂ (p_{A}CO₂) and that in the arterial blood (PaCO₂) of approximately 40 mm Hg (in normal humans). When one produces more CO₂ (e.g. as a result of fever or exercise), more CO₂ is carried to the lungs and one then has to breathe harder to wash out the extra CO₂ from the alveoli, and thus maintain the same equilibrium p_{A}CO₂, but if the CO₂ production stays normal, and one hyperventilates, then excess CO₂ is washed out of the alveoli and the p_{A}CO₂ falls. There are many scenarios in which we wish the inspired CO₂ to be greater than that which would normally come about physiologically. This heightened state of CO₂ in the system has many protective benefits. It often results in discomfort proportional to the increase in the PaCO₂ however, there is a very large safety range whereby the PaCO₂ in the body can double without known harm. When PaCO₂ increases acutely to greater than about 80 mmHg, it may affect consciousness but this effect is reversible on lowering the PaCO₂. Few other harmful effects are recognized.

One way to contribute to the pCO₂ levels of the organism can be by the delivery of one or more medicaments that are known to alter pH of the organism such as carbonic anhydrase inhibitors. Some examples of carbonic anhydrase inhibitors are Topiramate, Methazolamide, Dorzolamide or Acetazolamide. Carbonic anhydrase inhibitors can act as mild diuretics by reducing NaCl and bicarbonate reabsorption in the proximal tubule of the kidney. The bicarbonaturia will thus produce a metabolic acidosis. This mild acidosis has many potential benefits in mitigating SLOSH as described within. Anticipated acidic pH changes that would prove beneficial would be between about 7.30 and 7.40.

Another embodiment of elevating pCO₂ in the body of an organism can be a breathing circuit that maintains an elevated pCO₂. A circuit can maintain an estimated yet elevated end tidal pCO₂ by interposing one or more channels or tubes through which the individual breathes that causes a re-breathing of their previously exhaled breath. These channels allow a mixing of inhaled ambient gas and exhaled alveolar gas. The optimal amount of gas re-breathed can be determined by estimating the individual's weight in kilograms and multiplying it by a factor, such as 7, to arrive at an estimated tidal volume in cm³. In one embodiment a third of this volume can be added to the breathing circuit as dead space. This volume determines the predicted level of end tidal CO₂ to which the device will equilibrate. Alternatively an external source of CO₂ could be interposed to rapidly, and on demand, increase the percentage of inspired CO₂.

With respect to adding a dead space to increase rebreathing of previously exhaled gas, several paper or thin walled tubes or channels can extend away from the enclosed mouth and nose portion of the device and/or several regions can be placed sequentially along the channels or tubing as perforations or weakening points so as the individual will be able to tear, cut, or break off a predetermined amount of the tubing and thus precisely alter the remaining dead space of the circuit. Demarcations and identifiers placed along the channels/tubing can help the individual decide at which perforation or weakened zone to tear, cut, or remove. Again, these can be determined as follows: Tidal volume can be estimated by measuring one's weight in kilograms and multiplying by 7, the result would be in cm³ of tidal volume. To determine the amount of dead space to add to the outflow tract of the mask, one need only take the resultant tidal volume and add a corresponding percentage of the tidal volume (say 33%) to the outflow tract of the mask. Each incremental increase in dead space added to the outflow tract would cause an incremental increase in final pCO₂. For example, if the weight of the individual is 120kg then the estimated tidal volume would be 840 cm³. We would want the individual to re-breath a portion of that tidal volume equating to 33% of this which equals 277 cm³. This added volume of dead space would be expected to increase the pCO₂ by approximately 7-8 mm Hg.

In addition to the adjustable dead space, monitoring the end tidal CO₂ and driving the export valve to open or close to alter the source of the next inspired breath may be utilized in settings whereby precise knowledge of end tidal CO₂ may be required. For example, if an end tidal CO₂ desired range is 45 mm Hg, then upon noting the end tidal CO₂ being only 35 mm Hg, the valve would be directed to close requiring the individual to take the next breath from the adjustable dead space reservoir/tubing that a previous breath had been collected into. This expiration typically has 4-5% CO₂ within it allowing a greater inspired CO₂ on the next breath. A reservoir can act as a buffer to store extra CO₂ gas. Even when ventilation increases, the subject breaths the accumulated gas with elevated CO₂ allowing pCO₂ to rise to the desired level.

A circuit to maintain normal CO₂ can include a non-rebreathing valve, a source of fresh gas, a fresh gas reservoir and a source of gas to be inhaled, such as from the increased dead space region or a reservoir of higher concentration of CO₂. The method of controlling pCO₂ in a patient at a predetermined desired level can be provided comprising a breathing circuit/mask which is capable of increasing the CO₂ to enable an increase in cerebral blood flow and resultant cerebral blood pressure and volume. With increased cerebral blood flow, cerebral blood velocity, and intracranial pressure and volume there remains less space for intracranial tissues to move in relation to each other, thus brain pulsitility and SLOSH is diminished. This would require minimizing the intracranial volume of compressible or displaceable air/fluid/tissue structures. Although brain tissue is thought to be incompressible and fluid/blood is also relatively incompressible, the fluids may be able to be displaced through the vessels and between the cranium and the spinal canal, allowing to and fro movement of contents within the cranium and thus shearing forces between cells, concussive forces applied to structures, and absorption of blast wave energies. If either the elevated CO₂ has been implemented with a resultant increase in cerebral blood volume and/or there has been increased intracranial pressure by any means before a traumatic event, the brain and its components would be less prone to slosh around within the cranium and in relation to each individual component (thus better approximating elastic collisions). Further, if TBI were to occur despite the above restraining effects of the increased cerebral blood flow, an elevated CO₂ would even serve to optimize the healing environment of the brain tissue itself by reducing the systemic inflammatory response and maximizing flow of oxygen rich hemoglobin which is more capable of delivering its oxygen due to high levels of CO₂ through the reduction of oxygen affinity of the hemoglobin.

The human erythrocyte is very distensible and, as such, is particularly capable of absorbing energies imparted into it by suffering inelastic collisions. Mathematical analysis of Newton's Cradle shows that inelastic collisions absorb energy as acoustic, vibrational, heat and kinetic energy whereas elastic collisions serve to allow the forces to pass through without imparting as much energy. However, triggering an increase in pCO₂ in the blood and serum (resulting in erythrocytes pumping the CO₂ into the cytoplasm), can serve to cause nearly an immediate increase in bicarbonate created within the erythrocyte creating an osmotic swelling of fluid into the cell and reducing SLOSH absorption. Further, the walls of erythrocytes that have been exposed to higher levels of CO₂ have been shown to be less distensible; and also if swollen, they should facilitate more elastic collisions when forces are imparted into them. While not wishing to be bound by any one theory, it is likely that chloride shifts in response to CO₂ and bicarbonate movement will also serve to conformationally tighten the hemoglobin molecule, reducing energy absorption, and thereby better mitigating damage from SLOSH in the red blood cells and the tissues. Even after the mechanism that supplies extra CO₂ has been removed, it will take hours for the cells to equilibrate back to pre-hypercapnia levels. This would further serve to reduce force impartation into the brain or any erythrocyte perfused structures. The reversible swelling of the cells and the altered red cell membrane's distensibility would also serve to reduce the shear thinning capability that blood typically exhibits and again, this would serve to better approximate elastic collisions when forces are imparted.

In addition to increasing the volume within the cell one can also reversibly alter the vascular, molecular, or cell wall configuration within the organism to reduce SLOSH energy absorption. The configuration of the cell wall can be reversibly altered to increase membrane stability and decrease membrane fluidity. The addition of one or more of DHA and Magnesium oxide can be used to alter erythrocyte cell wall configuration. Typical DHA supplementations would be in the order of 50-3000 mg orally a day and MgO of 50-1000 mg orally a day. The configuration of hemoglobin can also be reversibly altered by altering one or more of pH (to a pH of about 7.0 to7.5), pCO₂ (to pCO₂ of about 25 to 80 mm Hg) or blood levels of 2,3- Bisphosphoglycerate (to about 6.0 to 1000 µmol/mL) within the organism to decrease hemoglobin elasticity and fluidity. 2,3- Bisphosphoglycerate levels may be increased by methods such as phosphate loading.

SLOSH absorption may also be reduced by reversibly increasing pressure or volume within the organs or cells of the organism. The intracranial volume and pressure can be reversibly increased by a device that reduces the flow of one or more outflow vessels of the cranium of said organism. One embodiment of such a device would compress the outflow vessels enough to cause an increase in venous resistance, yet not surpass arterial pressure of approximately 80 mm Hg. Intracranial volume can also be reversibly increased by increasing the pCO₂ in the arterial blood or by the delivery of one or more medicaments to facilitate an increase in intracranial volume or pressure including but not limited to Minocycline, insulin-like growth factor 1, Provera, and Vitamin A.

One aspect of the disclosure, therefore, encompasses embodiments of a compression device that when applied to the neck of a subject animal or human to reduce the likelihood of energy absorption to the brain through raising intracranial and intraocular volume and pressure by applying pressure to the outflow vasculature and/or cerebral spinal fluid of the brain. The result would be an increase in the structure's coefficient of restitution (r) by attaching a cinch or collar around the neck of the individual or organism. The compression device can be of any design including, but not limited to, a band or cord. Such a compression device could be worn preferably before, in anticipation of and during events with SLOSH and traumatic brain injury risks. The compression device of the disclosure further includes at least one protuberance or thickened region of the device that may be positioned over an underlying neck vein to apply a localized pressure thereto.

Embodiments of the collar of the disclosure, therefore, comprise a collar that can encircle completely or partially the neck of a subject animal or human, and is sized such that the collar can apply an external pressure over the regions of the neck overlying the internal jugular veins. It is contemplated that this pressure may be due to the internal dimension of the collar being less than the neck diameter resulting from the size of the collar or the elasticity thereof, or the result of decreasing the internal diameter of the collar by any method such as inflating the collar, a region thereof, or of at least one protuberance thereof. The external pressure applied to the internal jugular vein will result in a restriction of blood flow through the vein.

In particular, the collar of the device according to the disclosure, therefore, includes at least one protuberance, stud, thickened or expandable region directed inwardly and disposed on the surface of the collar that is proximal to the skin when applied to a neck so as to be positioned directly over a region of a neck vein. It is contemplated that the at least one protuberance, stud, thickened or expandable region can be a fixed protuberance or stud resistant to deformation when applying pressure to a neck, or may be enlarged by inflation of an inflation device connected thereto. An inflatable protuberance may be positioned over a neck vein and then inflated to apply pressure to the underlying blood vessel. In the alternative, the at least one protuberance may be non-inflatable but disposed on an inflatable collar.

It is further contemplated that the collar of the disclosure and/or inflatable protuberance(s) disposed thereon may be operably connected to an inflation means such as, but not limited to, a powered pump, or a hand-compressible pump whereby a liquid, air or a gas can be applied to the collar. In certain embodiments the collar may further comprise a pressure sensor operably linked to the inflation means whereby the degree of inflation of the collar and/or protuberance(s) thereof may be regulated as to the extent and duration of the pressure applied to an underlying neck vein.

It is also contemplated that the protuberance(s) of the collar of the disclosure may be configured to apply pressure to an area approximately the diameter of an internal jugular vein, greater than said diameter, and may be of any shape that can provide partial restriction of the blood flow through the neck vein, including a pointed protuberance, a stud, a thickened region of the collar, and the like.

The compression device may be of any material including, but not limited to, elastic materials. Elastic materials can be any material which when stretched will attempt to return to the natural state and can include one or more of textiles, films (wovens, non-wovens and nettings), foams and rubber (synthetics and natural), polychloroprene (e.g. NEOPRENE.RTM), elastane and other polyurethane- polyurea copolymerss (e.g. SPANDEX.RTM, LYCRA. RTM), fleece, warp knits or narrow elastic fabrics, raschel, tricot, milanese knits, satin, twill, nylon, cotton tweed, yarns, rayon, polyester, leather, canvas, polyurethane, rubberized materials, elastomers, and vinyl. There are also a number of elastic materials which are breathable or moisture wicking which may be preferable during extended wearing periods or wearing during periods of exercise. In addition the compression device could be partially constructed, coated, or constructed of one or more protecting materials such as Kevlar (para-aramid synthetic fibers), Dyneema (ultra-high-molecular-weight polyethylene), ceramics, or shear thickening fluids.

The device may encompass circumferentially the entire neck or just partially around the neck, yet still providing partial or total occlusion of one or more of the outflow vessels on the neck, specifically, but not limited to the internal and external jugular veins, the vertebral veins, and the cerebral spinal circulation. The device may encompass horizontally, the entire neck or just partially up and down the neck.

One embodiment of the compression device may be preformed for the user in a circular construct. This one size fits all style can have a cinch of sorts that allows one to conform the device to any neck size. Alternatively the compression device may have a first end and a second end which are connected by a fastener. A fastener may be a hook and ladder attachment, a hook and loop attachment, a snap, a button or any of a number of attachment mechanisms that would be known to one skilled in the art. A compression device with a fastener could have a breakaway release mechanism whereby the device can break open or apart at a predetermined force to prevent the collar from inadvertently being snagged or compressing too tightly. One quick release or automatic release embodiment would be the applying of small amounts of hook and ladder attachments within the circumferential ring which would shear apart upon too much force being applied to the compression device. Another embodiment of the device could fasten such that the user would be able to pull one end of the collar (like a choker collar for a dog) and the force exerted by the user effectually decreases the length or circumference of the device. When the desired neck compression is no longer needed (such as between football plays) the user could then release the compression by a second gentle tug or by a separate release mechanism also positioned on the device.

In an embodiment of the collar device according to the present invention, the protuberances that can apply a compressive pressure to an internal jugular vein are compressible pads or solid forms sized to apply pressure substantially only to internal jugular vein. It is contemplated that at least one pad or rigid form may be connected to one or both opposing ends of a resilient arcuate connector that conforms to a predetermined configuration such that the opposing ends of the connector may be displaced to allow the pad(s) or rigid form(s) thereon to be disposed on the neck to apply pressure to the underlying internal jugular vein.

The compression device may have one or more protuberances, or otherwise not be of consistent thickness or width. One such embodiment may have thicker protruding regions to be aligned with the internal jugular veins to preferentially apply a compressive pressure to these veins as the collar is tightened. Another embodiment may utilize inflatable protuberances as further described below.

The compression device may also have one or more monitoring, recording, and/or communicating devices attached or embedded. One such embodiment of the invention would be to embed a transceiver and/or receiver to allow communications between soldiers on a battlefield or even between coaches and players. Further, cardiac monitors could include heart rate or plethysmography monitors that could provide real time evaluation of cardiophysiology while the compression device is in place.

The compression device can also have a pocket or pouch attached depending on the height of the compression device used. Certainly, advertising can be imprinted or emblazoned onto the device. One such embodiment of the invention would have a wider segment of the collar on which to print a commercial design or brand name.

One other means of restricting blood flow within the neck vasculature would be to incorporate one or more segments of inflatable bladders within the collar to alter the circumference or pressure the collar is exerting. One such embodiment could utilize a bulb pump placed in connection to the bladders whereby the user would compress the bulb one or multiple times until the desired pressure of air or fluid is retained within the bladder of the collar. Another embodiment may utilize pressurized gas or fluid which is connected to the bladders. Another embodiment would have a pressure release valve in communication with the bladders such that once a predetermined pressure is reached within the bladder, any successive pumping actions would merely divert the air or fluid pressure to the ambient air or the pump itself would simply no longer inflate (an existing correlate example would be the historical "Reebok Pump"). An embodiment with a pressure release valve could prevent overinflation of bladders and allow for a very precise degree of pressure delivery to the vasculature.

Safely and reversibly increasing cerebral blood volume by any amount up to10 cm³ and pressure by any amount up to 70 mm Hg would serve to fill up the compliance of the cerebral vascular tree and thus reduce the ability to absorb external energies through SLOSH energy absorption. With the application of measured pressure to the neck, the cranial blood volume increases rapidly and plateaus at a new higher level. Moyer et al. ((1954) *Applied Physiol.* 7: 245) reported that cerebral arterial blood flow was not affected by obstructing the venous outflow of blood from the brain. The blood volume-venous pressure relationship shows a diminishing increase in volume with each increment of neck pressure over the range 40 to 70 mm Hg. The cranial blood volume increases from 10 to 30 per cent with this neck pressure (Kitano et al., (1964) J. Nuc. Med. 5: 613-625). The cerebral spinal fluid pressure responds on compression of the individual jugular veins. Jugular compression increases cerebral blood flow to a new plateau in as little as 0.5 seconds (Kitano et al., (1964) J. Nuc. Med. 5: 616; Gilland et al., (1969) Am. J. Roet. 106: 369).

This degree of cranial blood volume and pressure increase is advantageous in SLOSH mitigation. Although lesser cranial pressure and volume increases may still have beneficial effects, an increase of 3cm³ volume and 5 mm Hg is a baseline goal. However, if pressure is distributed along the length of the veins, much less pressure, for example, as little as 1-10 mm Hg is sufficient to increase flow resistance in the veins.

The safety of such a procedure of venous compression mirrors the 100 year old Quenkenstadt Maneuver. In this maneuver, "the compression of the neck does not interfere with arterial flow into the cranium. Although the venous jugular flow beneath the pressure cuff may be temporarily halted, the venous outflow from the cranium is never completely stopped, particularly from the anastomosis between the spinal vein and the basilar plexus and occipital sinuses which are incompressible. (Batson 0. V., (1944) Fed. Proc. 3: 139; Gregg & Andshipley (1944) Fed. Proc. 3: 144). In fact, there was no correlation between electroencephalographic (EEG) changes or changes in systolic arterial blood pressure occurring during jugular compression. Thus, neck compression of up to 70 mm Hg does not affect cardiac output, arteriolar blood pressure, pulse rate, or urine flow.

One aspect of the disclosure, therefore, encompasses embodiments of a device to reduce SLOSH energy absorption in an animal or human subject by reducing the flow of one or more neck veins by compressing at least one of said vessels, wherein the device can comprise a collar configured to at least partially encircle the neck of an animal or human subject, and at least one region inwardly directed to contact the neck when encircled by the collar, thereby applying a localized pressure to a neck vein.

In some embodiments of this aspect of the disclosure, the at least one region inwardly directed to contact the neck can be selected from the group consisting of: a protuberance, a stud, and a thickened region of the collar, and wherein the protuberance, the stud, and the thickened region of the collar can be rigid, semi-rigid, or inflatable.

In some embodiments of this aspect of the disclosure, the at least one region inwardly directed to contact the neck can be disposed on said collar to exert pressure in the area of an internal jugular vein when the neck of an animal or human subject is inserted in said collar.

In some embodiments of this aspect of the disclosure, the collar can be elastic.

In some embodiments of this aspect of the disclosure, the collar size and tension thereof can be adjustable.

In some embodiments of this aspect of the disclosure, the device can further comprise one or more breakaway release mechanisms.

In some embodiments of this aspect of the disclosure, the device can further comprise a monitoring device, a recording device, a communicating device, or any combination thereof.

In some embodiments of this aspect of the disclosure, the at least one region of the device inwardly directed to contact the neck of a subject can be formed by inflation of a region of the collar, and wherein the device optionally further comprises a pump to inflate the inflatable protuberance, and optionally a source of pressurized gas or fluid for inflation thereof.

In some embodiments of this aspect of the disclosure, the device can further comprise a release valve to regulate the pressure in said collar.

In some embodiments of this aspect of the disclosure, the device can further comprise a compressible pad or rigid form sized for applying pressure substantially only to a internal jugular vein when disposed against a neck surface, wherein said pad or form is disposed at one end of a resilient arcuate connector.

In some embodiments of this aspect of the disclosure, the device can further comprise a plurality of compressible pads or rigid forms sized for applying pressure substantially only to a internal jugular vein, wherein at least one pad or form is disposed at each opposing end of the resilient arcuate connector.

Another aspect of the disclosure encompasses embodiments of a method of increasing the intracranial pressure of an animal or human subject comprising: (i) encircling the neck of an animal or human subject with a collar, wherein said collar has at least one region inwardly directed to contact the neck of an animal or human subject; (ii) positioning the at least one region inwardly directed to contact the neck on a region of the neck overlying a neck vein carrying blood from the intracranial cavity of the subject; and (iii) applying pressure to the neck vein by pressing the at least one region inwardly directed to contact the neck onto the surface of the neck, thereby restricting blood flow egressing the intracranial cavity of the subject, thereby increasing the intracranial pressure of the subject.

In some embodiments of this aspect of the disclosure, the method can further comprise the step of increasing the pC02 of the intracranial cavity, thereby reducing the effect of intracranial sloshing on the blood of the animal or human subject.

In some embodiments of this aspect of the disclosure, the method can further comprise applying to the animal or human subject a device configured to receive exhaled breath from the subject and to recirculate the exhaled breath back to the subject, thereby increasing the pC02 of the intracranial cavity.

In some embodiments of this aspect of the disclosure, the device configured to receive exhaled breath from the subject and to recirculate the exhaled breath back to the subject can comprise a source of C02 and a means to deliver the C02 to the inhaled breath of the subject

In some embodiments of this aspect of the disclosure, the method can further comprise increasing the concentration of C02 in the inhaled breadth of the subject by providing an external supply of C02 to the recirculated breath of the subject.

The following examples are put forth so as to provide those of ordinary skill in the art with a complete disclosure and description of how to perform the methods and use the compositions and compounds disclosed and claimed herein. Efforts have been made to ensure accuracy with respect to numbers (e.g. , amounts, temperature, etc.), but some errors and deviations should be accounted for. Unless indicated otherwise, parts are parts by weight, temperature is in °C, and pressure is at or near atmospheric. Standard temperature and pressure are defined as 20 °C and 1 bar (1 atmosphere).

It should be noted that ratios, concentrations, amounts, and other numerical data may be expressed herein in a range format. It is to be understood that such a range format is used for convenience and brevity, and thus, should be interpreted in a flexible manner to include not only the numerical values explicitly recited as the limits of the range, but also to include all the individual numerical values or sub-ranges encompassed within that range as if each numerical value and sub-range is explicitly recited. To illustrate, a concentration range of "about 0.1% to about 5%" should be interpreted to include not only the explicitly recited concentration of about 0.1 wt% to about 5 wt%, but also include individual concentrations (*e.g.,* 1%, 2%, 3%, and 4%) and the sub-ranges (e.g., 0.5%, 1.1%, 2.2%, 3.3%, and 4.4%) within the indicated range. The term "about" can include ±1%, ±2%, ±3%, ±4%, ±5%, ±6%, ±7%, ±8%, ±9%, or ±10%, or more of the numerical value(s) being modified.

### EXAMPLES

### Example 1

*Materials and Methods:* Two groups of ten (total of 20) male Sprague-Dawley rats weighing between 350 and 400 grams were used. Animals were housed under 12 hour light/12 hour dark conditions with rat chow and water available *ad libitum.*

*Marmarou impact acceleration injury model in rats:* Anesthesia was induced and maintained with isoflurane using a modified medical anesthesia machine. Body temperature was controlled during the approximately 10 min. procedures using a homeothermic heating blanket with rectal probe, and adequate sedation was confirmed by evaluation of response to heel tendon pinch. The animals were shaved and prepared in sterile fashion for surgery, followed by subcutaneous injection of 1% lidocaine local anesthetic into the planned incision site. A 3 cm midline incision in the scalp was made, and periosteal membranes separated, exposing bregma and lambda. A metal disk 10 mm in diameter and 3 mm thick was attached to the skull with cyanoacrylate and centered between bregma and lambda.

The animal was placed prone on a foam bed with the metal disk directly under a plexiglas tube. A 450-g brass weight was dropped a single time through the tube from a height of 2 meters, striking the disk. The animal was then ventilated on 100% oxygen while the skull was inspected, the disk removed, and the incision repaired. When the animal recovered spontaneous respirations, anesthesia was discontinued and the animal was returned to its cage for post -operative observation. Buprenorphine was used for postoperative analgesia.

### Example 2

*Experimental protocol:* This work involved two groups, each consisting of 10 animals for a total of 20 animals. Two groups were utilized, a control injury group and an experimental injury group. In the experimental injury group the rats were fitted with a 15 mm wide collar, with two compressive beads designed to overlay the IJVs and was tightened sufficiently to provide mild compression of the veins without compromising the airway. The collar was then fixed in circumference with a Velcro fastener. The collar was left in position for three minutes prior to administrating experimental brain injury.

*Assessment of Intracranial Reserve Volume Intracranial Pressure (ICP) Measurement:* ICP was measured in five animals using the FOP-MIV pressure sensor (FISO Technologies, Quebec, Canada) as described by Chavko, *et al.* The head of the rat was shaved and prepped in sterile fashion for surgery. The rat was fixed in a stereotaxic apparatus (model962; Dual Ultra Precise Small Animal Stereotaxic Instrument, Kopf Instruments, Germany) and a 3 cm mid-line incision in the scalp was made. Periosteal membranes were separated, exposing both bregma and lambda. A 2 mm burr hole was drilled 0.9 mm caudal from bregma and 1.5 mm from the midline. The fiberoptic probe was then inserted to a depth of 3 mm into the cerebral parenchyma.

*Intraocular Pressure (IOP) Measurement:* IOP was measured in all animals usmg the TonoLab rebound tonometer (Colonial Medical Supply, Franconia, NH) as described in the literature. IOP measurements were taken after induction of anesthesia in all animals and a second time in the experimental group following application of the novel IJV compression device. Following application of the IJV compression device in the experimental injury group, IOP readings were taken every 30 secs while the compression device was in place. *Tissue Preparation and Imrnunhistochemical Labeling:* At 7 days post-injury all animals (n=20) were anesthetized and immediately perfused transcardially with 200 ml cold 0.9% saline to wash out all blood. This was followed by 4% paraformaldehyde infusion in Millings buffer for 40 mins. The entire brain, brainstem, and rostral spinal cord were removed and immediately placed in 4% paraforrnaldehyde for 24 hours. Following 24 hours fixation, the brain was blocked by cutting the brainstem above the pons, cutting the cerebellar peduncles, and then making sagittal cuts lateral to the pyramids. The resulting tissue, containing the corticospinal tracts and the mediallenmisci, areas shown previously to yield traumatically injured axons, was then sagitally cut on a vibratome into 50 micron thick sections.

The tissue underwent temperature controlled microwave antigen retrieval using previously described techniques. The tissue was pre-incubated in a solution containing 10% normal serum and 0.2% Triton X in PBS for 40 mins. For amyloid precursor protein (APP) labeling, the tissue was incubated in polyclonal antibody raised in rabbit against beta APP (#51-2700, Zymed, Inc., San Francisco, CA) at a dilution of 1:200 in 1% NGS in PBS overnight. Following incubation in primary antibody, the tissue was washed 3 times in 1% NGS in PBS, then incubated in a secondary anti-rabbit IgG antibody conjugated with Alexa 488 fluorophore (Molecular Probes, Eugene, OR), diluted at 1:200 for two hours. The tissue underwent a final wash in 0.IM phosphate buffer, and then was mounted using an antifade agent and cover-slipped. The slides were sealed with acrylic and stored in the dark in a laboratory refrigerator.

*Fluorescent Microscopy and Image analysis:* The tissue was examined and images acquired using a Olympus AX70 fluorescence microscope system (Olympus; Tokyo, Japan). Ten digital images were obtained from the tissue of each animal and images were then randomized. Individual injured axons were independently counted and data was stored in a spreadsheet (Microsoft Corp., Redmond, WA). Differences between group means were determined using paired t-tests and considered significant if the probability value was less than 0.05. *Stereological Quantification of axonal injury:* A stereo logical method was used to determine an unbiased estimate of the number of APP positive axons per cubic mm in the corticospinal tract and medial lemniscus. The optical fractionator technique utilizing a Stereoinvestigator 9.0 (MBF Bioscience, Inc., Williston, VT) and a Olympus AX70 microscope with 4x and 40x objectives was performed. Sagittal APP stained specimens were examined with low magnification and regions of interest were drawn incorporating the corticospinal tract and medial lemniscus. The software then selected random 50 micron counting frames with depth of 15 microns, and APP positive axons were marked. The volume of the region of interest (ROI) was determined using the Cavalieri method, the volume of the sum of the counting frames was calculated, the sum total of injured axons within the counting frames was calculated, and an estimate of the number of APP positive axons per cubic mm was calculated.

### Example 3

### Assessment of Intracranial Reserve

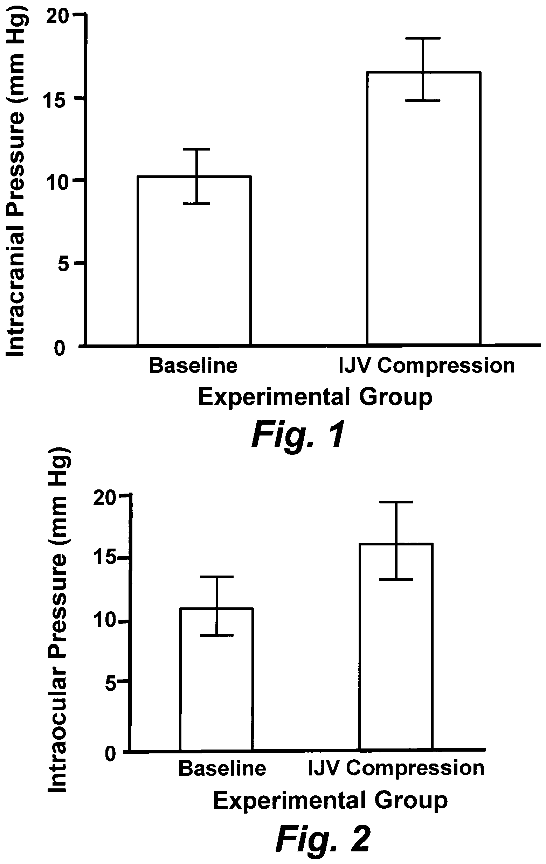

*Volume Intracranial Pressure (ICP) Measurement:* ICP was assessed both prior to and after application of the IJV compression device. The baseline ICP was 10.23 ± 1.68 mm Hg and was increased to 16.63 ± 2.00 mm Hg following IJV compression (Fig. 1: p<0.01). Notably, this increase of greater than 30% from baseline occurred within seconds following IJV compression. Intraocular Pressure (IOP) Measurement: IOP measurements were taken both before and after application of the IJV compression device, similar to ICP recordings. The baseline IOP was 11.18 ± 2.27 mm Hg and was elevated to 16.27 ± 3.20 mm Hg following IJV compression (Fig. 2: p<0.01).

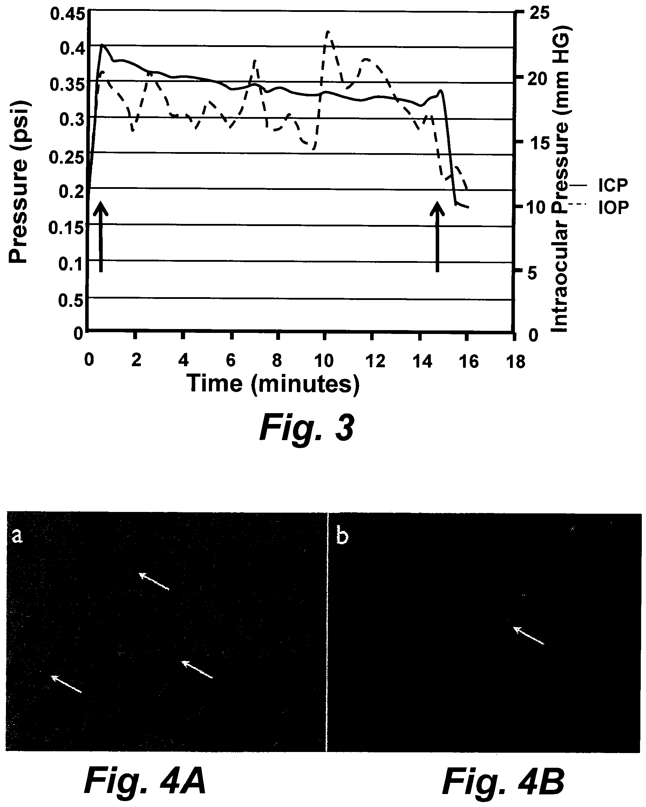

The increase of 31% seen in IOP following IJV compression is strikingly similar to that seen in ICP following IJV compression, both in magnitude and rapidity of response (Fig. 3).

*TBI- Impact Acceleration Model:* None of the animals died from the head trauma. Animals tolerated collar application without any observed untoward effects for the duration of the experiment. Specifically, there were no outward or visible signs of discomfort, intolerance, or respiratory difficulty. All recovered without complication and exhibited normal behavioral and feeding habits up until the day of sacrifice. At necropsy, the brains were grossly normal in appearance.

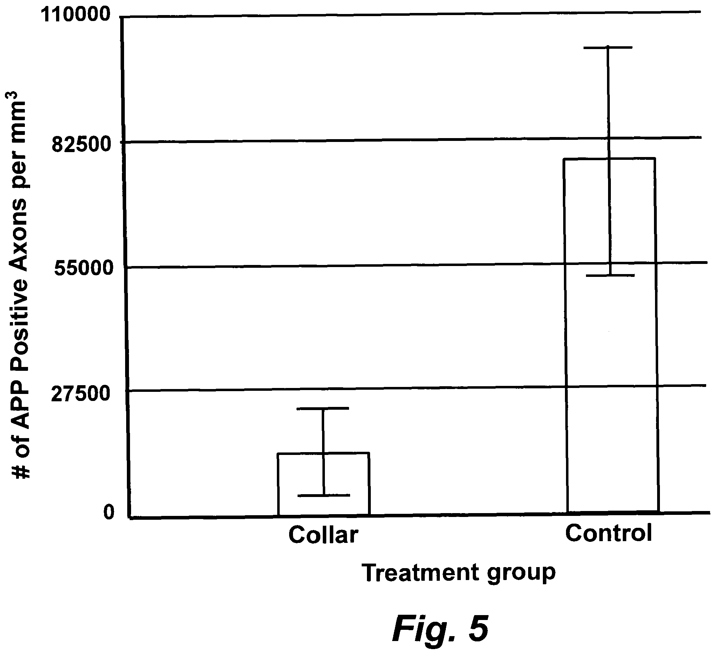

*Stereologic Analysis of APP Positive Axons:* To determine the density of injured axons in the corticospinal tracts and medial lenmisci, the stereo logical optical fractionator method was

used. Compared to the normal anatomy found in previous experiments with sham animals, control animals without the collar demonstrated focal labeling of APP within many swollen contiguous and terminal axon segments, consistent with impaired axoplasmic transport in traumatic axonal injury. Following microscopic digital image acquisition from multiple areas within the corticospinal tract and medial lenmisci from multiple tissue slices, counting of APP positive axons in animals who received the IJV compression collar demonstrated much fewer APP positive axons, at a frequency much more similar to sham animals, compared to those undergoing injury without IJV compression (Figs. 4A and 4B). These abnormal axons demonstrated typical morphological characteristics of traumatic injury, primarily swelling and disconnection. By qualitative analysis, the experimental group showed (m ± sd) 13,540 ± 9808 vs. 77,474 ± 25,325 (p<O.OI) APP positive axons/ mm3 in the control group (Fig. 5).

### Example 4

Two groups of 10 adult male Sprague-Dawley rats were subjected to an impact acceleration traumatic brain injury. Prior to the injury, the experimental group had application of a 15 mm wide cervical collar, which had two compressive beads over the internal jugular veins (IJVs). The control group had the experimental injury only. Intracranial pressure (ICP) and intraocular pressure (IOP) were measured before and after IJV compression to assess collar performance. All rats were sacrificed after a 7-day recovery period, and brainstem white matter tracts underwent fluorescent immunohistochemical processing and labeling of beta amyloid precursor protein (APP), a marker of axonal injury. Digital imaging and statistical analyses were used to determine if IJV compression resulted in a diminished number of injured axons.

### Example 5

All animals survived the experimental paradigm and there were no adverse reactions noted following application of the collar. In the experimental group, IJV compression resulted in an immediate and reversible elevation of ICP and IOP, by approximately 30%, demonstrating physiologic changes secondary to collar application. Most notably, quantitative analysis showed 13,540 APP positive axons in the experimental group versus 77,474 in the control group , a marked reduction of greater than 80%.

Using a standard acceleration-deceleration impact laboratory model of mild TBI, a reduction of axonal injury following IJV compression as indicated by immunohistochemical staining of APP was shown. IJV compression reduces Slosh-mediated brain injury by increasing intracranial blood volume and reducing the compliance and potential for brain movement within the confines of the skull.

### Example 6

*Internal versus External Brain Protection:* Compression of the IJV for 3 min prior to head trauma led to physiological alterations in intracranial compliance, as evidenced by modest increases in ICP and IOP, while simultaneously and markedly reducing the pathologic index of primary neuronal injury in the standardized rat model of TBI. Reduction in brain volume compliance could prevent the differential motions between the cranium and the brain that lead to energy absorption and neuronal primary and secondary injuries. These pathological changes include axonal tearing that disrupt axoplasmic transport resulting in axonal swelling and activation of the apoptotic cascades, as evidenced in this model by a statistically significant reduction in APP counts of injured axons.

In the animal model of the present disclosure, applying the collar increased ICP and IOP by 30% and 31%, respectively. The effect of compression of jugular veins on ICP is clinically well known. The Queckenstadt test is used to indicate the continuity of CSF between the skull and spinal cord. In this test, ICP is increased by compression of the IJVs while the CSF pressure is measured in the spine through a lumbar puncture. Increases in ICP have also been shown to occur with placement of tight fitting neck stabilization collars that likely compress the IJVs. Compression of the IJVs, which can occur when wearing shirts with tight collars or neckties, has also been shown to increase IOP. Notably, only mild compressive pressure is required to partially occlude the IJVs as they are a low pressure system. As the inflow of cerebral arterial blood continues after partial cerebral venous outlet obstruction, the intracerebral and venous pressure increases until the jugular venous resistance is overcome or the blood drainage is redirected to other venous channels. In either case there is a reduction in intracranial compliance and a modest increase in ICP.

The immunohistochemical assay used in the studies of the present disclosure is specific for axonal damage and results in a reliable range of measured damaged neurons. In addition, the Marmarou model of acceleration-deceleration injury is an accepted and well-studied methodology by which to quantify the extent of TBI. The reduction in damaged axons, as evidenced by a marked reduction in APP counts, in the experimental group with the IJV compression device is highly statistically significant (p<0.01). Additionally, the change in ICP was measured after applying the collar in five rats. The results show that every study rat had a reduction in axonal injury greater than the 95% confidence interval of the control group.

## Claims

1. A collar device comprising a plurality of rigid forms on a resilient arcuate connector;
wherein the collar device is sized to partially encircle the neck of a human subject,
wherein at least one of the plurality of rigid forms is disposed at each opposing end of the resilient arcuate connector to partially or totally occlude one or more of the outflow vessels of the cranium when the device is worn around the neck by the subject; and
wherein one or more of the outflow vessel is an internal jugular vein, and wherein the at least one of the plurality of rigid forms is sized to apply pressure substantially only to the internal jugular vein.

## Patentansprüche

1. Kragenvorrichtung, aufweisend mehrere starre Formen auf einem elastischen bogenförmigen Verbinder;
wobei die Kragenvorrichtung so bemessen ist, dass sie den Hals eines menschlichen Subjekts teilweise umfasst;
wobei mindestens eine der mehreren starren Formen an jedem gegenüberliegenden Ende des elastischen bogenförmigen Verbinders angeordnet ist, um ein oder mehrere der Ausflussgefäße des Schädels teilweise oder vollständig zu verschließen, wenn die Vorrichtung von dem Subjekt um den Hals getragen wird; und
wobei ein oder mehrere der Ausflussgefäße eine innere Jugularvene aufweisen, und wobei die mindestens eine der mehreren starren Formen so bemessen ist, dass sie im Wesentlichen nur auf die innere Jugularvene Druck ausübt.

## Revendications

1. Un dispositif de collier comprenant une pluralité de formes rigides sur un raccord arqué résilient ;
dans lequel le dispositif de collier est dimensionné pour encercler partiellement le cou d'un sujet humain,
dans lequel au moins une de la pluralité de formes rigides est disposée à chaque extrémité opposée du raccord arqué résilient pour occlure partiellement ou totalement un ou plusieurs des vaisseaux de sortie du crâne lorsque le dispositif est porté autour du cou par le sujet ; et
dans lequel un ou plusieurs des vaisseaux de sortie sont une veine jugulaire interne, et
dans lequel la au moins une de la pluralité de formes rigides est dimensionnée pour appliquer une pression sensiblement uniquement sur la veine jugulaire interne.
